# FASCICULE DE BREVET EUROPEEN

(11) **EP 2 183 016 B1**
(45) Date de publication et mention de la délivrance du brevet: **29.11.2017**
(21) Numéro de dépôt: 08803278.4
(22) Date de dépôt: 27.08.2008
(51) Int. Cl.: A61M 39/28, A61M 5/142

(54) **PINCE DE SERRAGE POUR TUBULURE SOUPLE, POMPE MUNIE DE MOYENS POUR OUVRIR UNE TELLE PINCE ET SET DE PERFUSION MUNI D'UNE TELLE PINCE**
KLEMME FÜR EINEN SCHLAUCH, PUMPE MIT MITTEL ZUR ÖFFNUNG EINER SOLCHEN KLEMME UND MIT EINER SOLCHEN KLEMME AUSGESTATTETES INFUSIONSKIT
CLAMP FOR A FLEXIBLE TUBE, PUMP PROVIDED WITH MEANS FOR OPENING SUCH A CLAMP, AND INFUSION KIT PROVIDED WITH SUCH A CLAMP

(30) Priorité: 04.09.2007 FR 0757337
(43) Date de publication de la demande: 12.05.2010
(73) Titulaire: Fresenius Vial SAS, 38590 Brézins (FR)
(72) Inventeur: TRAVERSAZ, Philippe, 38140 Saint-blaise Du Buis (FR)
(74) Mandataire: Vièl, Frédérique
(86) Numéro de dépôt international: PCT/EP2008/061252
(87) Numéro de publication internationale: WO 2009/030625

(56) Documents cités:
- EP-A- 0 186 509
- WO-A-98/48872
- WO-A-03/041787
- WO-A-03/063945
- US-A1- 2006 081 797

## Description

L'invention concerne une pince de serrage pour une tubulure souple, notamment une tubulure pour perfusion, constituée d'une bande de matière plastique essentiellement en forme de U, l'extrémité libre de la première branche du U étant cintrée, pour former la tête de la pince, vers l'extrémité libre de la deuxième branche, appelée dos, de sorte qu'en position fermée, l'extrémité libre du dos se trouve en appui sous l'extrémité libre de la tête, tandis qu'en position ouverte, l'extrémité libre du dos se trouve à distance au-dessus de l'extrémité libre de la tête, la pince ayant une paire d'ouvertures pour permettre à une tubulure souple de s'étendre à travers la pince et d'être supportée par celle-ci et deux saillies opposées pour obturer la tubulure souple en la comprimant lorsque la pince est en position fermée. L'invention concerne également une pompe munie de moyens pour ouvrir et fermer une pince selon l'invention, un set de perfusion muni d'une pince selon l'invention et une combinaison d'une pompe selon l'invention et d'un set de perfusion selon l'invention.

Le document EP 1 218 055 A1 décrit une telle pince, couramment appelée pince (ou clamp) escargot. Il décrit également une telle pompe pour perfusion, un tel set de perfusion ainsi qu'une telle combinaison d'une pompe pour perfusion et d'un set de perfusion. La pompe décrite dans ce document est munie de moyens pour fermer la pince et de moyens pour ouvrir la pince qui lorsqu'une pression est exercée par un élément d'actionnement d'une pompe de perfusion pour ouvrir ou fermer la pince. Les moyens pour fermer la pince ont la forme d'un bombement formé sur la face extérieure de l'extrémité sur lequel roule au moins une partie d'un levier basculant de la pompe pour perfusion.

Le document WO 00/44 434 A1 décrit également une pince de serrage pour tubulure souple conformément au préambule de la revendication 1. Cette pince de serrage possède des ouvertures pour la tubulure souple qui s'étendent axialement à travers le bord latéral de la pince et qui sont positionnées chacune à proximité d'une partie de bande étroite par comparaison au reste de la bande. Pour renforcer la partie de bande étroite, le document propose qu'une au moins des parties de bande étroites s'étende latéralement vers l'extérieur au delà du second bord latéral du reste de la bande.

Les document WO 2003/041787 A2, WO 98/48872 et EP 0 186 509 A2 décrivent tous une pince de serrage selon le préambule de la revendication 1 et un set selon le préambule de la revendication 14. Dans le document WO 2003/041787, une rondelle excentrée est prévue dans une pompe pour ouvrir et fermer la pince. Pour cela, l'enveloppe cylindrique de la rondelle excentrée appuie sur la tête de la pince pour libérer la prise entre la tête et l'extrémité du dos. En faisant tourner la rondelle dans le sens antihoraire, la tête de l'extrémité libre est dépliée latéralement pour ouvrir la pince. La pince du document WO 98/48872 A1 peut être ouverte en faisant pivoter manuellement l'extrémité de tête de sorte à supprimer l'engagement des deux surfaces de chevauchement. Pour ouvrir la pince de EP0186509 A2, le médecin doit appuyé avec le pouce sur la tête de la pince pour faire pivoter de façon élastique l'extrémité cintrée portant la tête

La pince de serrage du document EP 1 218 055 A1 qui peut être fermée et ouverte par un moyen d'actionnement de la pompe à perfusion a considérablement augmenté la sécurité de fonctionnement de tels dispositifs.

Cependant, en position fermée, ces clamps escargot peuvent être ouvertes par inadvertance en poussant sur l'extrémité libre de la tête, extrémité qui retient en position poussée l'autre extrémité de la pince.

L'invention a pour objectif d'améliorer encore la sécurité de fonctionnement et de faciliter les manipulations nécessaires pour mettre en place une perfusion.

Cet objectif est atteint pour une pince selon le préambule en ce que des moyens de blocage sont prévus pour empêcher d'écarter l'extrémité libre de tête de l'extrémité de dos lorsque la pince est en position fermée.

Ceci peut être facilement réalisé par exemple lorsque les moyens de blocage sont constitués par un tenon situé sur l'extrémité de tête et par un évidement situé sur l'extrémité de dos, le tenon et l'évidement étant disposés de telle sorte qu'en position fermée de la pince, le tenon pénètre dans l'évidement, un élément d'actionnement étant de préférence prévu sur l'extrémité de tête. Une fois le tenon logé dans l'évidement, il est impossible d'ouvrir la pince par une simple pression sur l'extrémité de tête, comme cela est le cas avec les pinces connues dans l'état de la technique. En effet, pour pouvoir écarter l'extrémité de tête afin de libérer l'extrémité de dos, il faut aussi bien appuyer sur le dos pour faire sortir le tenon de l'évidement qu'écarter l'extrémité de tête. Ce double mouvement est en soi facile à réaliser, mais nécessite de coordonner ces deux mouvements, de sorte qu'une ouverture involontaire de la pince est pratiquement exclue. Lors d'une simple pression sur le dos de la pince, le tenon sort de l'évidement, mais rentre à nouveau dans celui-ci lorsque la pression cesse s'il n'a pas été écarté. La pince reste donc fermée.

Conformément à l'invention, un insert est prévu dans une des ouvertures de la pince pour recevoir l'extrémité de la tubulure souple, l'insert étant solidaire de la pince. Cet insert est de préférence réalisé en matière plastique dure et de forme cylindrique. Du fait qu'il est solidaire de la pince, il ne peut pas être déplacé dans le sens de la tubulure. Il est de préférence inséré dans l'ouverture située dans le fond du U formant la pince. Il protège l'extrémité de la tubulure souple sortant de la pince de serrage et qui traverse cette ouverture.

Dans un développement de l'invention, l'insert est muni d'un embout pour fixer une autre tubulure souple. L'insert peut donc servir de raccord entre deux tubulures souples, éventuellement entre deux tubulures souples de qualités différentes, par exemple une tubulure très souple passant à travers une pompe à doigts et une autre moins souple qui n'est pas soumise aux mêmes sollicitations.

Un autre développement de l'invention consiste à munir l'insert sur son pourtour extérieur d'une rainure dans laquelle pénètrent les deux bords opposés de l'ouverture de la pince qui elle-même peut être munie d'un logement pour recevoir l'insert. L'insert est maintenu dans la pince par emboîtement. Si l'ouverture de la pince a la forme d'une fente qui s'étend sur environ 180° dans le fond du U formant la pince, l'élément de guidage bien que maintenu dans la pince peut basculer dans cette fente autour d'un axe passant par le logement. S'il est de forme cylindrique, il peut également pivoter.

Il est conforme à l'invention de prévoir une rainure longitudinale sur la face extérieure du dos de la pince. Cette rainure sur le milieu du dos de la pince permet de guider une came d'actionnement placée sur un levier basculant de la pompe de perfusion lors de l'ouverture ou la fermeture de la pince.

L'invention concerne également une pompe pour perfusion munie d'une porte pouvant être ouverte et fermée afin de placer ou d'enlever une tubulure souple munie d'une pince conforme à l'invention, la pompe étant munie de moyens pour ouvrir ou fermer une pince située à l'intérieur, moyens qui comprennent un levier pivotant muni d'au moins une came de tête pour écarter l'extrémité de tête de l'extrémité de dos. Conformément à l'invention, le levier est muni de moyens pour débloquer les moyens de blocage de la pince. Il est en outre préférable que la pompe soit munie de moyens pour fermer la pince lorsqu'on ouvre la porte pour éviter tout risque de débit libre lorsqu'on retire la tubulure de la pompe.

Ceci est facilement réalisé par exemple lorsque les moyens de déblocage sont constitués par au moins une came de dos, la came de tête et la came de dos étant disposées sur l'axe de pivotement du levier de telle sorte que lors de l'actionnement du levier pour ouvrir la pince, la came de dos appuie tout d'abord sur le dos de la pince jusqu'à déverrouiller les moyens de blocage de la pince et la maintient dans cette position, puis la came de tête écarte l'extrémité libre de tête jusqu'à laisser un passage pour l'extrémité de dos, la came de dos relâche ensuite la pression sur le dos de la pince et enfin, la came de tête relâche l'extrémité de tête. Le levier permet donc d'ouvrir ou de fermer la pince, l'ouverture s'effectuant en exerçant une pression sur le dos de la pince puis en écartant de celle-ci l'autre extrémité afin que les deux extrémités s'éloignent l'une de l'autre. Ainsi, non seulement on sort le tenon de l'évidement, mais en plus on décale le tenon par rapport à l'évidement de sorte qu'il ne peut plus retomber dedans. Pour la fermeture, une simple pression sur le dos de la pince est suffisante pour que le tenon de la pince rentre dans l'évidement. Il est bien entendu également possible de refaire à l'envers le mouvement d'ouverture décrit précédemment.

Dans une variante de l'invention, le levier est placé dans la porte de la pompe. Pour utiliser le levier aussi bien pour ouvrir et fermer la pince que pour ouvrir et fermer la porte, il est possible de le munir en outre d'un crochet pouvant coopérer avec un galet situé sur le boîtier de la pompe pour maintenir la porte fermée.

Dans une variante de l'invention, l'opération peut être effectuée par un système motorisé synchrone avec l'ouverture et la fermeture de la porte.

Dans un mode de réalisation privilégié de l'invention, la porte est munie d'un élément de rappel qui tend à déplacer le crochet en position fermée dès que les moyens d'ouverture et de fermeture de la pince ont placé la pince en position ouverte. Ainsi, on est sûr que la porte est bien fermée dès que le levier est lâché après fermeture de la porte (et donc que la pince est ouverte). De même, en cas de soulèvement non intentionnel du levier sans volonté d'ouverture de la porte, celui-ci sera ramené en position fermée sans déclencher la fermeture de la pince tant qu'un certain point de pivotement n'aura pas été atteint.

Il est également utile de prévoir un support sur le boîtier de la pompe pour encliqueter l'insert de la pince.

L'invention concerne également un set pour perfusion constitué d'une tubulure souple passant à travers une pince conforme à l'invention.

Il est avantageux dans ce contexte de relier deux tubulures souples de qualités différentes l'une à l'autre au moyen d'un insert solidaire de la pince.

Enfin, l'invention concerne une combinaison d'une pompe pour perfusion conforme à l'invention et d'un set de tubulure souple pour perfusion conforme à l'invention.

L'invention est décrite ci-dessous à l'aide d'exemples de réalisation présentés sur les figures qui montrent :
- Figure 1: une pince de serrage conforme à l'invention vue en perspective ;
- Figures 2: la pince de serrage de la figure 1 (a) en position fermée, (d) en position ouverte et (b, c) dans des positions intermédiaires;

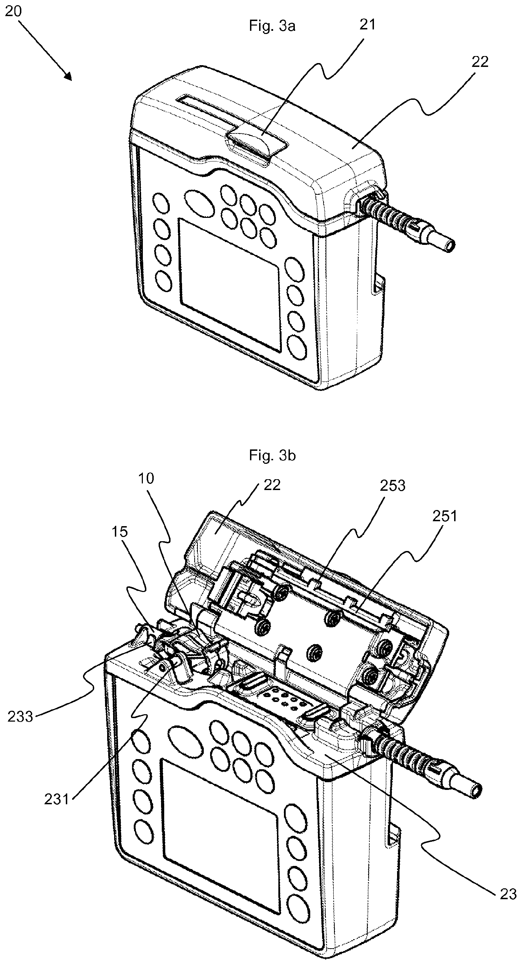

- Figures 3: des vues en perspective d'une pompe conforme à l'invention avec (a) la porte fermée et (b) la porte ouverte ;

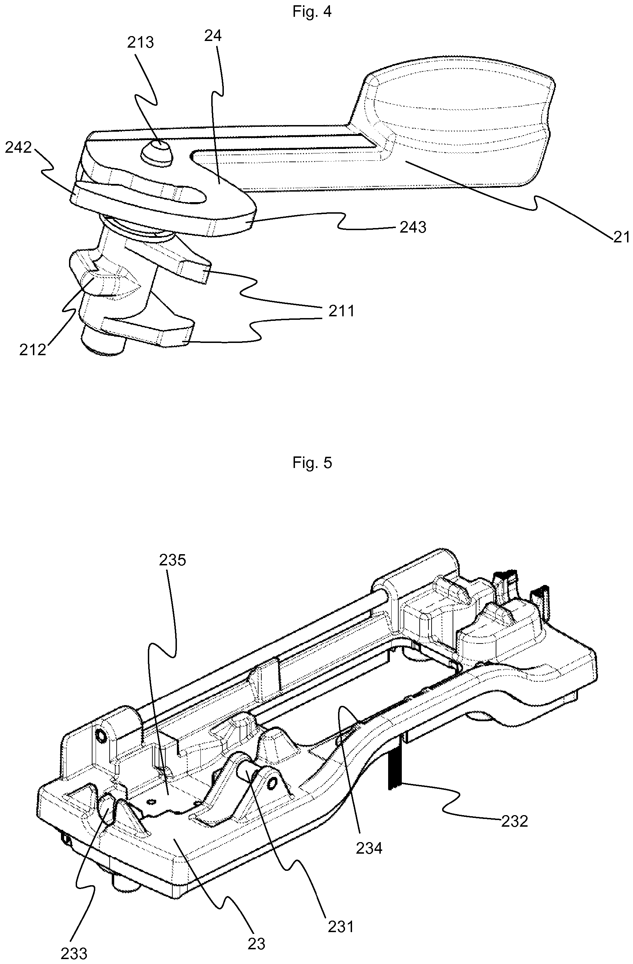

- Figure 4: une vue en perspective du levier de la pompe conforme à l'invention ;
- Figure 5: une vue de la partie du boîtier de la pompe située sous la porte lorsque celleci est fermée ;
- Figures 6: une représentation de la pompe lorsque le levier est en position ouverte (a) en coupe à travers les moyens de fermeture de la porte et (b) des moyens de déblocage de la pince ;
- Figures 7 et 8: des vues semblables aux figures 6, le levier étant progressivement rabattu ;
- Figures 9: des vues semblables aux figures 6, le levier étant totalement rabattu ;
- Figure 10: une vue en coupe transversale de la porte et de la face supérieure du boîtier de la pompe au moment où les moyens de déblocage entrent en contact avec le dos de la pince lorsqu'on rabat la porte ;

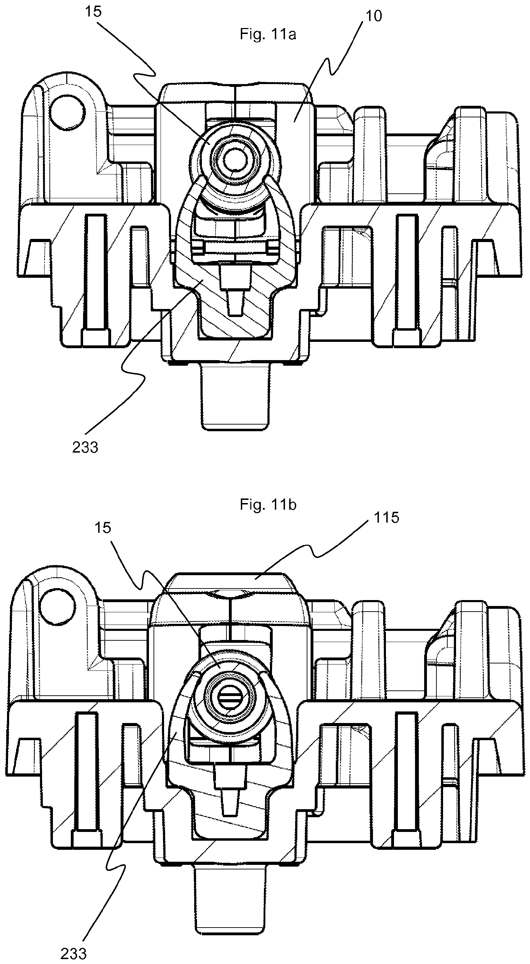

- Figure 11: des vues (a) avant et (b) après encliquetage de l'insert dans son support.

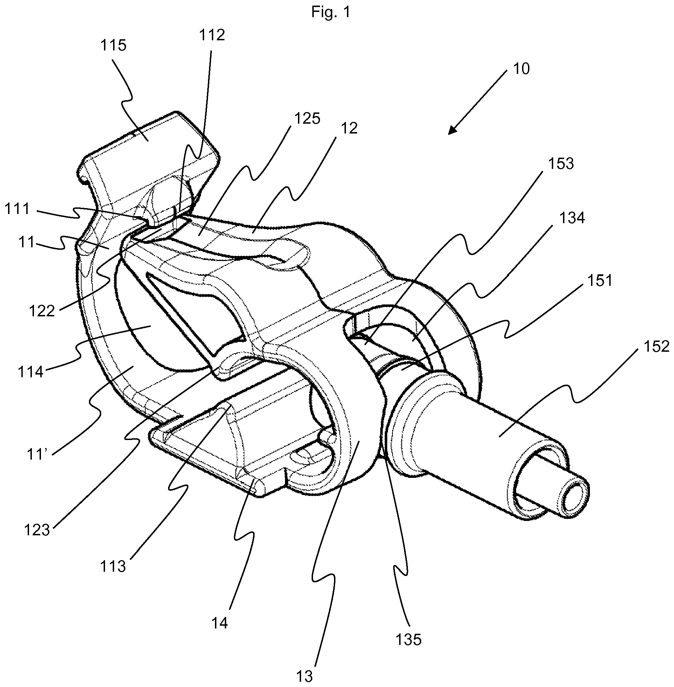

La figure 1 montre une pince de serrage (10) pour une tubulure souple pouvant être indéfiniment ouverte et refermée. Cette pince, de type clamp escargot, est constituée par une bande en matière plastique ayant une forme générale en U. La branche inférieure (11') du U a son extrémité cintrée vers le haut en direction de la branche supérieure (12). L'extrémité libre (111) de la branche inférieure (11) se prolonge par un élément d'actionnement (115). La branche supérieure (12) est appelée dos de la pince tandis que la partie cintrée (11) de la branche inférieure est appelée tête de la pince. Sans contrainte extérieure, l'extrémité libre (121) du dos (12) de la pince se trouve au-dessus de l'extrémité libre (111) de la tête (11). En position fermée, l'extrémité libre (121) du dos (12) se trouve sous l'extrémité libre (111) de la tête (11) plaquée contre elle sous l'effet de ressort de l'étrier formant le fond (13) du U.

La branche inférieure (11') et le dos (12) sont munis chacun d'une saillie (113, 123) servant, lorsque la pince est fermée, à pincer la tubulure qui la traverse. Deux ouvertures (134, 114) sont pratiquées dans le fond (13) et dans la tête (11) de la pince pour laisser passer la tubulure à travers la pince.

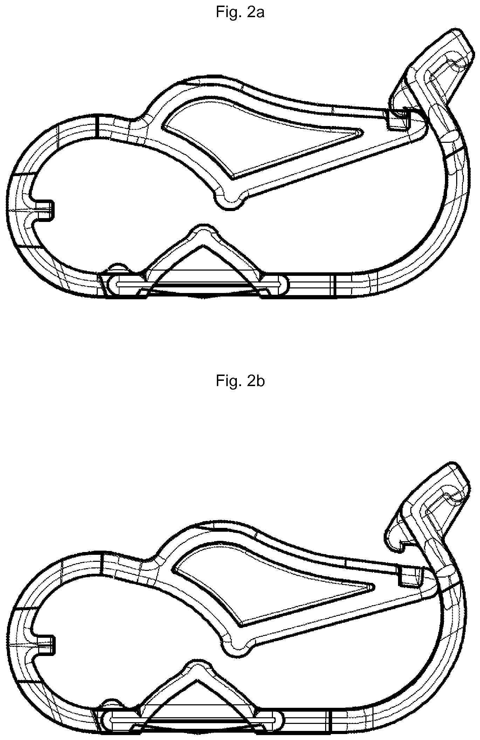

Pour ouvrir les pinces de l'état de la technique, il faut exercer une pression sur la tête de sorte à l'écarter de l'extrémité libre du dos, la pression s'exercant dans une direction sensiblement parallèle à la surface de chevauchement des deux extrémités. La pince (10) de l'invention se distingue de celles de l'état de la technique par la présence de moyens de blocage qui empêchent d'écarter involontairement l'extrémité libre (111) de la tête (11) de l'extrémité libre (121) du dos (12) lorsque la pince est en position fermée. Ces moyens de blocage sont constitués d'une part d'un tenon (112) situé sur l'extrémité libre (111) de la tête (11) et d'autre part d'un évidement (122) situé sur l'extrémité libre (121) du dos (12). Le tenon (112) et l'évidement (122) sont disposés sur leur branche respective de telle sorte qu'en position fermée de la pince, le tenon (112) pénètre dans l'évidement (122). Lorsque la pince est en position fermée, le tenon (112) et l'évidement (122) viennent en buté l'un contre l'autre si on essaie d'écarter l'extrémité (111) tête de l'extrémité (121) de dos selon un mouvement sensiblement perpendiculaire à la direction de pénétration du tenon. En d'autres termes, les surfaces de butée du tenon (112) et de l'évidement (122) sont sensiblement perpendiculaires à la surface de chevauchement des deux extrémités (111, 121). La pince est ainsi verrouillée. La figure 2a illustre cette situation.

Pour ouvrir la pince, il faut tout d'abord exercer une pression sur le dos (12) pour le déplacer vers le centre de la pince afin de faire sortir le tenon (112) de l'évidement (122). La figure 2b illustre cette position. Si cette pression est supprimée, le dos se relève sous l'effet de ressort de l'étrier formant le fond (13) du U, le tenon retourne dans l'évidement et la pince reste fermée.

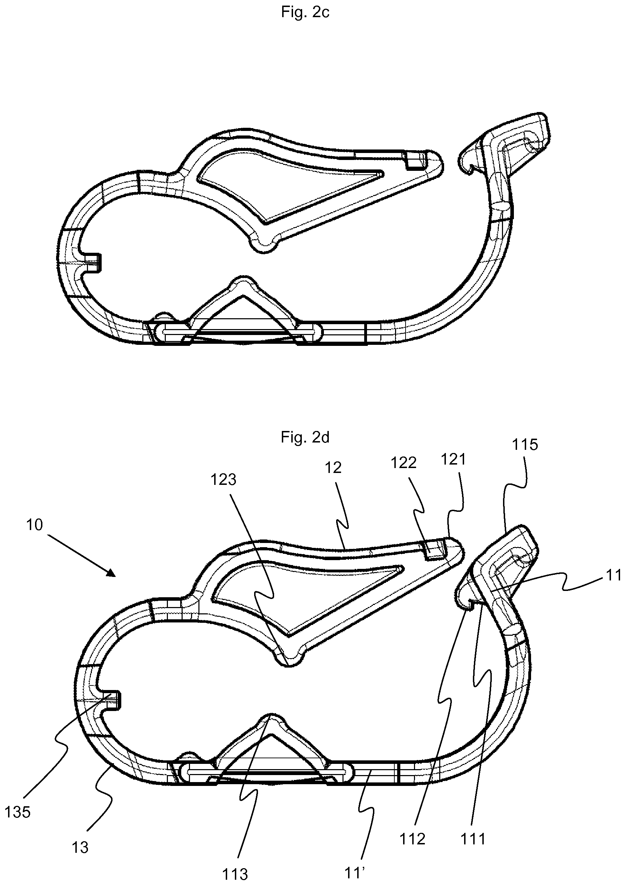

Lorsque le dos est suffisamment poussé vers l'intérieur de la pince, au-delà de la position fermée de la pince, de sorte que le tenon (112) se trouve hors de l'évidement (122), une pression peut être exercée sur l'extrémité libre (111) de la tête (11) pour l'écarter de l'extrémité libre (121) du dos jusqu'à ce que cette dernière puisse passer devant l'extrémité libre (111) de la tête (11) sans être bloquée. La pince se trouve dans la position illustrée à la figure 2c.

Tout en maintenant l'extrémité (111) de tête écartée, la pression exercée sur le dos (12) est supprimée. Le dos (12), sous l'effet de ressort du fond (13) de la pince, s'écarte de la branche inférieure (11'), son extrémité (121) passant devant l'extrémité de tête (111) écartée. La pression exercée pour écarter l'extrémité de tête peut maintenant être supprimée. La pince se trouve en position ouverte montrée à la figure 2d. Dans cette position, les deux saillies (113, 123) n'exercent plus de pression sur la tubulure et le fluide peut s'écouler normalement.

Grâce à ces moyens de blocage, il faut pour ouvrir la pince aussi bien appuyer sur le dos (12) de la pince et qu'écarter en même temps l'extrémité de tête (111) selon deux mouvements de direction sensiblement perpendiculaires. Ce double mouvement est facile à effectuer, mais comme il faut coordonner les deux mouvements, cela exclue pratiquement tout risque d'ouverture involontaire de la pince. Ces deux mouvements ne doivent pas être absolument perpendiculaires l'un à l'autre, il suffit qu'ils soient suffisamment inclinés pour empêcher un écartement en force provoquant le déplacement de l'extrémité libre du dos vers le centre de la pince sans qu'une pression ne soit exercée sur le dos. Ce principe est traduit ici par le terme « sensiblement » perpendiculaire.

Pour fermer la pince (10), il est possible d'appuyer simplement sur le dos (12) en direction de la branche inférieure (11'). L'extrémité de dos (121) glisse alors sur l'élément d'actionnement (115) incliné prolongeant l'extrémité de tête (111) et l'écarte suffisamment pour se procurer un passage. Dès que l'extrémité de dos (121) a dépassé l'extrémité de tête (111), celle-ci (111) retourne dans sa position initiale et bloque le dos (12).

Il est également possible de refaire à l'envers la même procédure que pour l'ouverture de la pince.

Lorsque la pince (10) de l'invention est placée dans une pompe, il est nécessaire de munir celle-ci de moyens pour débloquer la pince (10) afin de l'ouvrir lorsque la porte est fermée, ou de la fermer lorsque l'on ouvre la porte. Ceci est nécessaire afin d'éviter un écoulement libre ("free-flow") lors de l'ouverture ou de la fermeture de la porte (11), et ceci, quel que soit le stade de fermeture de la porte (11). De manière générale, la pince (10) doit être ouverte lorsque la porte (22) de la pompe (20) est fermée et être fermée lorsque la porte (22) de la pompe (20) est ouverte. Les pompes destinées à utiliser des clamps escargot sont donc en général munies de moyens pour ouvrir la pince lorsqu'on ferme la porte de la pompe et inversement pour fermer la pince lorsqu'on ouvre la porte.

Pour cela, la pompe (20) est munie d'un levier pivotant (21) placé en général dans la porte (22). Le levier (21) est muni sur son axe de rotation (213) d'au moins une came, ici une paire de came (211), pour écarter l'extrémité de tête (111) selon un mouvement sensiblement parallèle aux surfaces de chevauchement des extrémités, afin de laisser un passage à l'extrémité de dos (121).

Dans le cas présent, cette paire de cames de tête (211) ne peut cependant pas écarter l'extrémité de la tête, car celle-ci est retenue contre l'extrémité de dos par le tenon (112) qui pénètre dans l'évidement (122).

Le levier (21) est donc muni d'une seconde came (212), dite came de dos, pour appuyer sur le dos (12) de la pince afin de faire sortir préalablement le tenon (112) hors de l'évidement (122). La paire de cames de tête (211) et la came de dos (212) sont disposées sur l'axe du levier (21) de telle sorte que lorsque le levier est rabattu contre la porte (22), la came de dos (212) appuie d'abord sur le dos (12) avant que la paire de cames de tête (211) ne commence à écarter l'extrémité de tête (111).

Dans l'exemple de réalisation présenté ici, le levier (21) de l'invention est muni en outre d'un crochet (24) qui coopère avec un galet pivotant (231) placé sur la partie (23) de la pompe qui se situe sous la porte (22) lorsque celle-ci est fermée. Le levier (21) sert donc non seulement à ouvrir ou fermer la pince (10) de l'invention placée dans la pompe, mais également à ouvrir ou à fermer la porte (22) de la pompe.

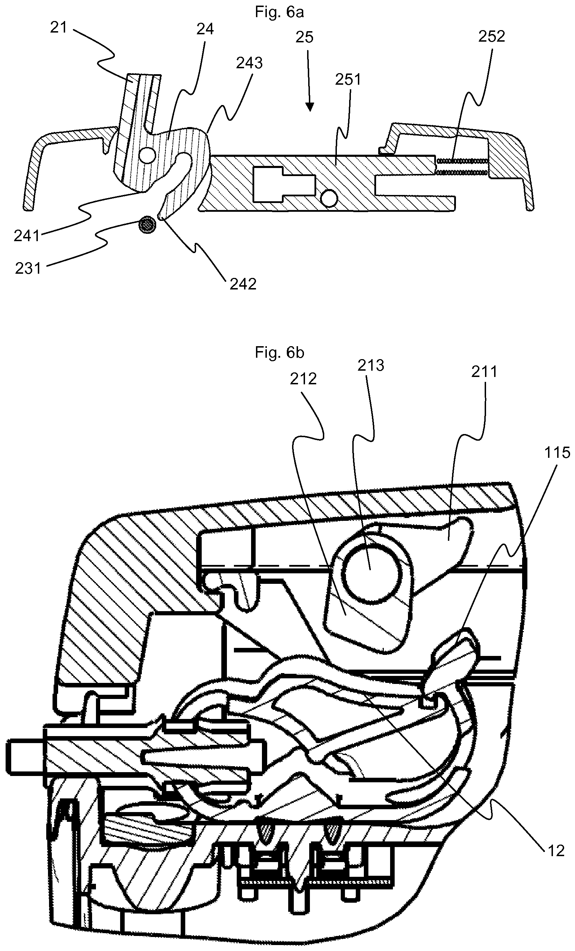

Les figures 6 à 9 montrent le déroulement de l'ouverture de la pince au cours de la fermeture de la porte.

Si le levier (21) est rabattu contre la porte alors que celle-ci est ouverte, il n'est pas possible de la rabattre entièrement contre le boîtier (23) de la pompe, car le crochet (24) vient buter contre le galet (231). Il est donc nécessaire de soulever le levier (21) pour fermer la porte.

Si le levier (21) est redressé, il n'est pas non plus possible de finir de fermer la porte, car la tête (241) du crochet vient buter contre le galet (231) lorsque la porte est rabattue. Il faut donc à ce moment-là commencer à rabattre le levier (21) contre le boîtier (23) de la pompe pour pouvoir verrouiller la porte en position fermée. La pointe (242) du crochet (24) saisit alors le galet (231) et glisse en dessous entraînant dans son mouvement la porte.

Lorsque le levier (21) est rabattu vers la porte (22), la pointe (242) passe sous le galet (231) et provoque tout d'abord un déplacement de l'axe de pivotement (213) du levier (21) en direction du boîtier (23) de la pompe, entraînant avec lui la porte. Il provoque également un mouvement de pivotement des cames (211 et 212) qui vont pouvoir agir sur la pince (10) pour l'ouvrir une fois que la porte sera verrouillée.

Si la pince (10) est ouverte, la came de dos (212) appuie sur le dos (12) de la pince (10) et provoque tout d'abord sa fermeture. On se trouve dans la situation présentée aux figures 6

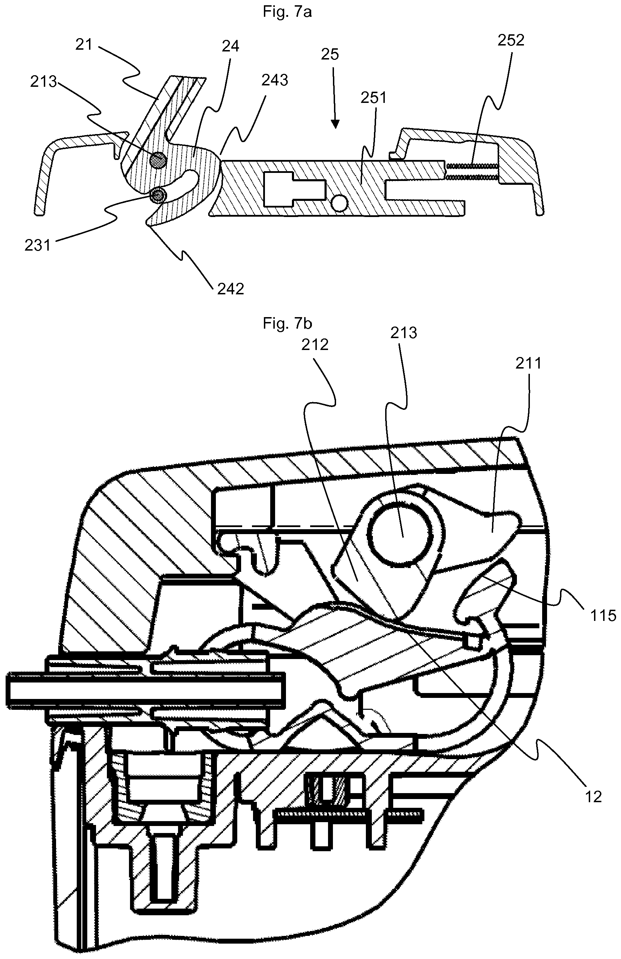

En continuant de rabattre le levier (21), la came de dos (212) continue à appuyer contre le dos (12) tout en pivotant. Le dos (12) est poussé vers l'intérieur de la pince (10) au-delà de la position fermée et le tenon de blocage (112) sort de l'évidement (122). C'est la position représentée aux figures 7.

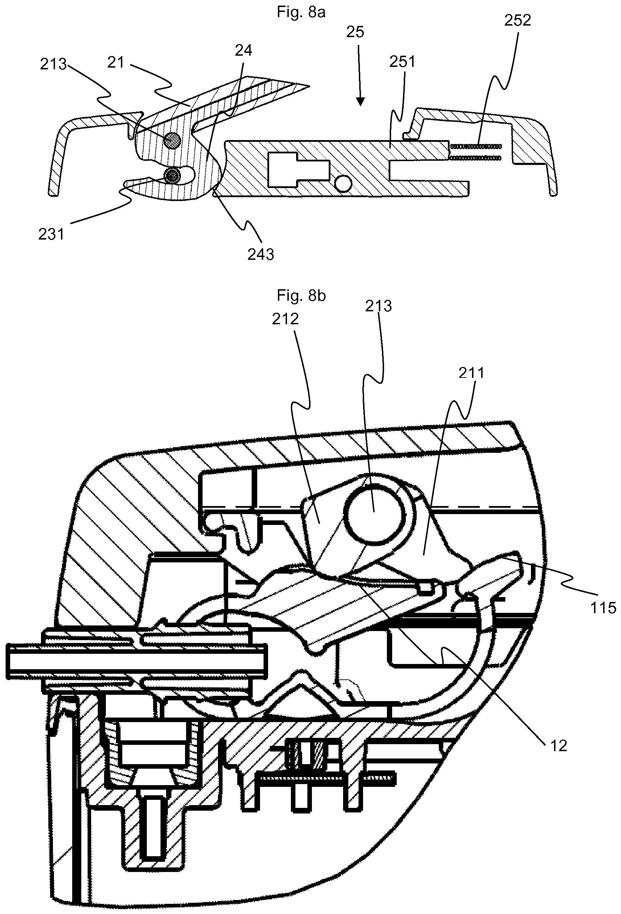

En continuant de faire pivoter le levier (21), la came de dos maintient en la diminuant lentement sa pression sur le dos (12) de la pince (10) tandis que la paire de cames de tête (211) entre en contact avec l'élément d'actionnement (115) de la tête (11) et écarte son extrémité libre (111) de l'extrémité de dos (112). Les deux extrémités (111, 121) sont donc chacune dans leur position extrême telle que représenté aux figures 8.

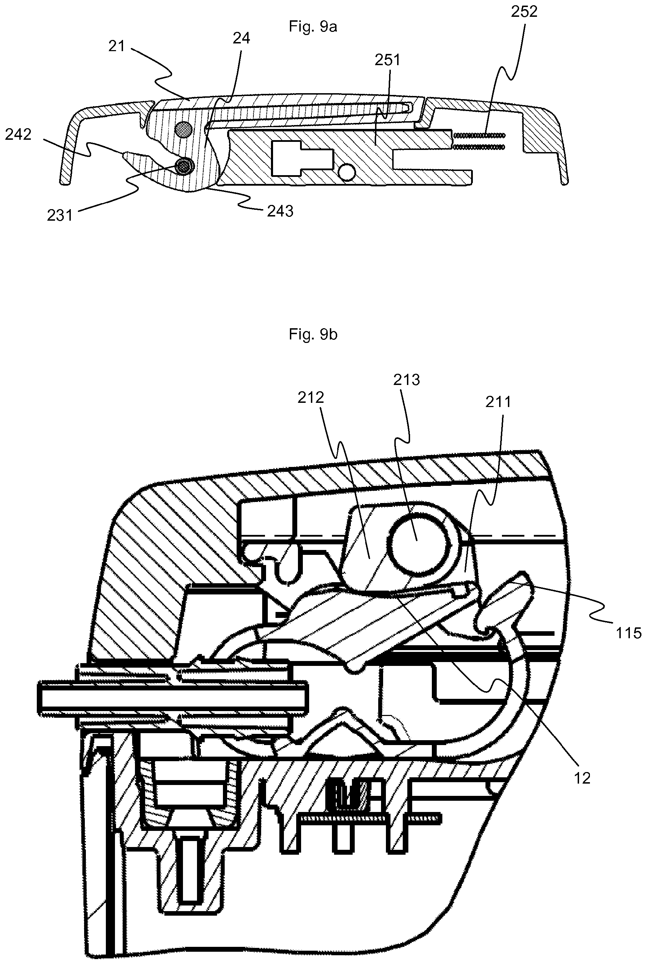

En poursuivant le mouvement de rabat du levier (21), on provoque le relâchement de la pression exercée par la came de dos (212) contre le dos (12) et la remontée en position ouverte de cette branche, puis, en dernier, le relâchement de la pression exercée sur l'élément d'actionnement (115). Enfin, la paire de cames de tête (211) passe au-delà de l'élément d'actionnement (115) et se glisse sous l'extrémité libre de tête (111). Le levier (21) est maintenant entièrement rabattu, la pince (10) ouverte et la porte (22) fermée et verrouillée dans cette position par le crochet (24). Les figures 9 illustrent cette situation.

Si maintenant on soulève le levier (21), la paire de cames de tête (211) va tout d'abord écarter l'extrémité de tête (111) pour laisser suffisamment de place pour le passage de l'autre extrémité (121). La came de dos appuie sur le dos (12) jusqu'à ce que son extrémité (121) soit passée devant l'extrémité de tête (111) écartée, toujours retenue par la paire de cames de tête (211). La paire de cames de tête (211) cesse ensuite d'appuyer sur l'élément d'actionnement (115) et l'extrémité de tête (111) retrouve sa position normale. Enfin, la came de dos (212) cesse à son tour d'appuyer sur le dos (12) qui remonte jusqu'à ce que son extrémité (121) vienne buter contre l'extrémité de tête, le tenon (112) pénétrant dans l'évidement (122). Le crochet (24) n'est plus en prise avec le galet (231) et la porte peut être ouverte. La pince (10) est à nouveau fermée.

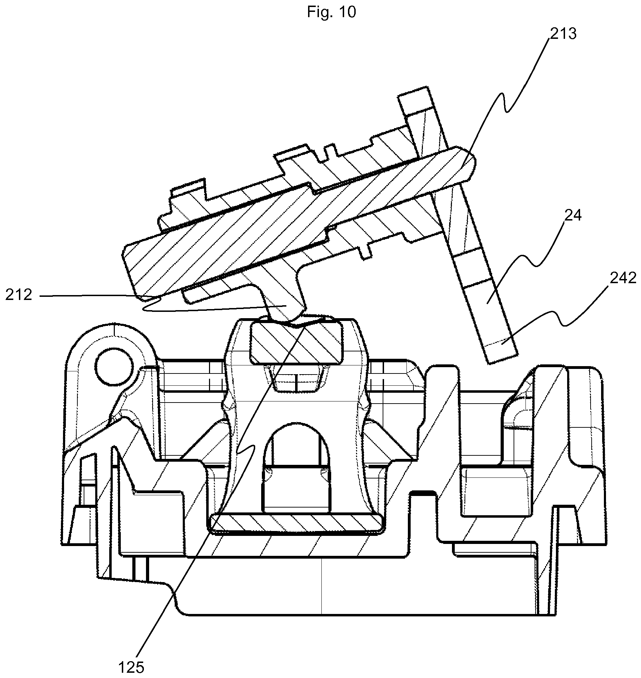

Pour guider la came de dos (212), il est préférable de munir le dos (12) de la pince d'une rainure de guidage (125). Cela évite que le dos (12) de la pince glisse et se positionne de travers lorsque la came de dos (212) appuie dessus. La figure 10 montre cette coopération.

Afin d'éviter que la porte ne soit ouverte par un mouvement involontaire, un dispositif de rappel (25) est prévu dans la porte (22). Ce dispositif de rappel (25) est constitué essentiellement d'une plaque mobile (251) poussée par un ressort (252) contre la face extérieure du crochet (24). Cette plaque (251) peut se déplacer en translation contre l'effet du ressort (252) si le levier (21) est soulevé. Tant que le levier (21) n'a pas dépassé un certain point dans son mouvement de levée, la plaque (251) appuie contre la face extérieure du fond (243) du crochet (24) et force le levier (21) à revenir en position rabattue contre la porte (22). Le point de non-retour correspond au moment où les cames de tête (211), qui au départ sont sous la tête (11), quittent l'extrémité de tête (111) pour passer sur l'élément d'actionnement (115) de la tête. En d'autres termes, tant que ce point de non-retour n'est pas dépassé, la pince (10) est toujours ouverte, le crochet (24) est toujours en prise avec le galet (231) et la porte (22) est rabattue. La plaque mobile (251) est munie d'ergots (253) qui viennent se loger sous des butées (234) placées sur le boîtier (23) de la pompe, lorsque la plaque subit le mouvement de translation sous l'effet du ressort. La porte (22) est donc non seulement verrouillée par le crochet (24) en prise avec le galet (231), mais également par les ergots (253) retenus par les butées (234).

De plus, des capteurs (232) peuvent être placés au niveau des butées (234) pour détecter la bonne position des ergots (253) témoignant de la bonne fermeture de la porte. Le signal fourni par le détecteur peut être utilisé pour commander la pompe (20).

Pour assurer un bon positionnement de la pince (10) dans la pompe (20), la pince (10) est munie d'ailes de positionnement (14) qui pénètrent dans des logements prévus à cet effet dans le boîtier (23). La forme de la pince (10) et du logement (235) correspondant interdisent un positionnement à l'envers de la tubulure dans la pompe.

Il est par ailleurs préférable d'immobiliser la pince (10) dans la pompe. Pour cela, un insert (15) est placé dans l'ouverture (134) située dans le fond en U de la pince. Cet insert (15) présente une gorge (151) circulaire sur sa périphérie. L'ouverture (134) présente dans le prolongement de la tubulure un logement circulaire (135) pour recevoir la gorge (151), la longueur du logement correspondant sensiblement à la largeur de la gorge (151). Ainsi, l'insert est bloqué en translation dans l'ouverture (134) de sorte qu'il ne peut pas se déplacer par rapport à la pince (10) dans le sens longitudinal de la tubulure ni le long de l'ouverture (134). Cependant, il peut légèrement basculer autour d'un axe de pivotement passant par les logements (135) pratiqués dans l'ouverture (134) ce qui laisse une certaine souplesse à l'ensemble tubulure / pince.

Cet insert (15), de préférence en matière plastique dure de forme cylindrique, protège l'extrémité de la tubulure souple sortant de la pince (10).

L'insert (15) se prolonge de part et d'autre de la gorge (151) par des embouts (152, 153). Des tubulures de qualités différentes peuvent être fixées à ces embouts (152, 153).

L'embout extérieur (152) peut coopérer avec un support d'insert (233) placé sur le boîtier (23) de la pompe. Ce support (233) est un canal cylindrique dans lequel peut s'encliqueter l'embout extérieur (152). Cet encliquetage provoque un « clic » lors de l'installation de la pince dans le boîtier. Si jamais, la pince n'est pas entièrement introduite dans son logement, et que l'embout extérieur (152) n'est pas correctement encliqueté dans son support (233), il est prévu dans la porte une nervure qui viendra appuyer sur l'embout extérieur (152) lors de la fermeture de la porte (22) jusqu'à ce qu'il se soit correctement encliqueté dans son support (233). Ainsi, au plus tard lors du premier verrouillage de la porte, la pince sera correctement immobilisée dans la pompe.

Références :
- 10: Pince
11 Tête de pince
11' Branche inférieure de la pince
111 Extrémité de tête
112 Tenon
113 Saillie
114 Ouverture
115 Élément d'actionnement
12 Branche supérieure (dos)
121 Extrémité de dos
122 Évidement
123 Saillie
125 Rainure de guidage
13 Fond
134 Ouverture de fond
135 Logement insert
14 Ailes de positionnement
15 Insert
151 Gorge
152 Embout extérieur
153 Embout intérieur
- 20: Pompe
21 Levier
211 Paire de cames de tête
212 Came de dos
213 Axe de rotation
22 Porte
23 Partie du boîtier recouverte par la porte lorsque celle-ci est fermée
231 Galet
232 Capteur
233 Support d'insert
234 Butées pour ergots
235 Logement pince
24 Crochet
241 Tête de crochet
242 Pointe de crochet
243 Fond du crochet
25 Dispositif de rappel
251 Plaque mobile
252 Ressort
253 Ergots

## Revendications

1. Pince de serrage (10) pouvant être ouverte et refermée pour une tubulure souple, notamment une tubulure pour perfusion, constituée d'une bande de matière plastique essentiellement en forme de U, l'extrémité libre (111) de la première branche (11') du U étant cintrée, pour former la tête (11) de la pince, vers l'extrémité libre (121) de la deuxième branche (12), appelée dos, de sorte qu'en position fermée, l'extrémité de dos (121) du dos (12) se trouve en appui sous l'extrémité libre (111) de la tête (11), tandis qu'en position ouverte, l'extrémité libre (121) du dos (12) se trouve à distance au-dessus de l'extrémité libre (111) de la tête (11), la pince (10) ayant une paire d'ouvertures (114, 134) espacées pour permettre à une tubulure souple de s'étendre à travers la pince (10) et d'être supportée par celle-ci et deux saillies (113, 123) opposées pour obturer la tubulure souple en la comprimant lorsque la pince est en position fermée, des moyens (115) étant prévus pour écarter l'extrémité de tête (111) de l'extrémité de dos (121) en exerçant une pression sur l'extrémité de tête (111) parallèlement aux surfaces de chevauchement des deux extrémités afin d'ouvrir la pince lorsqu'elle est en position fermée, des moyens de blocage (112, 122) étant prévus pour empêcher d'écarter l'extrémité de tête (111) de l'extrémité de dos (121) lorsque la pince (10) est en position fermée lorsque seule une pression est exercée sur l'extrémité de tête (111), les moyens de blocage étant constitués par un tenon (112) situé sur l'extrémité de tête (111) et par un évidement (122) situé sur l'extrémité de dos (121), le tenon (112) et l'évidement (122) étant disposés de telle sorte qu'en position fermée de la pince, le tenon (112) pénètre dans l'évidement (122), un élément d'actionnement (115) étant de préférence prévu sur l'extrémité de tête (111), **caractérisée en ce que** le tenon (112) et l'évidement (122) sont munis chacun d'une surface de butée qui coopèrent ensemble de sorte que le tenon et l'évidement viennent en buté l'un contre l'autre si on essaie d'écarter l'extrémité (111) tête de l'extrémité (121) de dos selon un mouvement sensiblement perpendiculaire à la direction de pénétration du tenon, les surfaces de butée du tenon (112) et de l'évidement (122) étant sensiblement perpendiculaires à la surface de chevauchement des deux extrémités (111, 121) de sorte que pour ouvrir la pince, il faut tout d'abord exercer une pression sur le dos (12) pour le déplacer vers le centre de la pince afin de faire sortir le tenon (112) de l'évidement (122).

2. Pince (10) selon la revendication précédente, **caractérisée en ce qu'**un insert (15) est prévu dans une des ouvertures (134) de la pince (10) pour recevoir l'extrémité de la tubulure souple, l'insert (15) étant solidaire de la pince (10).

3. Pince (10) selon la revendication 2, **caractérisée en ce que** l'insert (15) est muni d'un embout (152) pour fixer une autre tubulure souple.

4. Pince (10) selon la revendication 2 ou 3, **caractérisée en ce que** l'insert (15) est muni sur son pourtour extérieur d'une rainure (151) dans laquelle pénètrent les deux bords opposés de l'ouverture (134) de la pince (10).

5. Pince (10) selon l'une des revendications précédentes, **caractérisée en ce qu'**une rainure (125) longitudinale est prévue sur la face extérieure du dos (12).

6. Set pour perfusion constitué d'une tubulure souple passant à travers une pince (10) selon les revendications 1 à 5.

7. Set selon la revendication 6, **caractérisé en ce que** deux tubulures souples de qualités différentes sont reliées l'une à l'autre au moyen d'un insert (15) solidaire de la pince (10).

8. Combinaison d'une pompe (20) pour perfusion et d'un set de tubulure souple pour perfusion selon l'une des revendications 6 ou 7, dans laquelle la pompe pour perfusion (20) est munie d'une porte (22) pouvant être ouverte et fermée pour placer ou enlever une tubulure souple munie d'une pince (10) selon l'une des revendications 1 à 5, la pompe étant munie de moyens pour ouvrir ou fermer une pince (10) qui comprennent un levier (21) pivotant muni d'au moins une came de tête (211) pour écarter l'extrémité de tête (111) de l'extrémité de dos (121) d'une pince (10) située dans la pompe, **caractérisée en ce que** le levier (21) est muni de moyens (212) pour débloquer les moyens de blocage (112, 122) de la pince (10).

9. Combinaison selon la revendication précédente, **caractérisée en ce que** la pompe de perfusion est munie de moyens (211, 212) pour fermer la pince (10) lorsqu'on ouvre la porte (22).

10. Combinaison selon la revendication 8 ou 9, **caractérisée en ce que** les moyens de déblocage sont constitués par une came de dos (212), la came de tête (211) et la came de dos (212) étant disposées sur l'axe de pivotement (213) du levier (21) de telle sorte que lors de l'actionnement du levier (21) pour ouvrir la pince, la came de dos (212) appuie tout d'abord sur le dos (12) de la pince (10) jusqu'à déverrouiller les moyens de blocage (112, 122) de la pince (10) et la maintient dans cette position, puis la came de tête (211) écarte l'extrémité de tête (111) jusqu'à laisser un passage pour l'extrémité de dos (121), la came de dos (212) relâche ensuite la pression sur le dos (12) de la pince (10) et enfin, la came de tête (211) relâche l'extrémité de tête (111).

11. Combinaison selon l'une des revendications 8 à 10, **caractérisée en ce que** le levier (21) est placé dans la porte (22) de la pompe (20).

12. Combinaison selon l'une des revendications 8 à 11, **caractérisée en ce que** le levier (21) est muni en outre d'un crochet (24) pouvant coopérer avec un galet (231) situé sur le boîtier (23) de la pompe pour maintenir la porte (22) fermée.

13. Combinaison selon la revendication précédente, **caractérisée en ce que** la porte (22) est munie d'un élément de rappel (25) qui tend à déplacer le crochet (24) en position fermée dès que les moyens d'ouverture et de fermeture (211, 212) de la pince (10) ont placé la pince (10) en position ouverte.

14. Combinaison selon l'une des revendications 8 à 13, **caractérisée en ce qu'**un support (233) est prévu sur le boîtier (23) de la pompe (20) pour encliqueter l'insert (15) de la pince (10).

## Patentansprüche

1. Klemme (10), die geöffnet und wieder geschlossen werden kann, für einen flexiblen Schlauch, insbesondere einen Infusionsschlauch, die aus einem im Wesentlichen U-förmigen Kunststoffstreifen besteht, wobei das freie Ende (111) des ersten Schenkels (11') des U in Richtung des freien Endes (121) des als Rücken bezeichneten zweiten Schenkels (12) gebogen ist und so den Kopf (11) der Klemme bildet, damit in der geschlossenen Stellung das Rückenende (121) des Rückens (12) unter dem freien Ende (111) des Kopfs (11) anliegt, während sich das freie Ende (121) des Rückens (12) in der geöffneten Stellung in einem Abstand über dem freien Ende (111) des Kopfs (11) befindet, wobei die Klemme (10) ein Paar beabstandete Öffnungen (114, 134) aufweist, damit ein flexibler Schlauch durch die Klemme (10) verlaufen und von ihr gehalten werden kann, sowie zwei gegenüberliegende Vorsprünge (113, 123) zum Absperren des flexiblen Schlauchs durch Zusammendrücken, wenn sich die Klemme in der geschlossenen Stellung befindet, wobei Mittel (115) vorgesehen sind, um das Kopfende (111) vom Rückenende (121) zu entfernen, indem Druck auf das Kopfende (111) parallel zu den Überlappungsflächen der beiden Enden ausgeübt wird, damit die Klemme geöffnet wird, wenn sie sich in der geschlossenen Stellung befindet, wobei Arretiermittel (112, 122) vorgesehen sind, um ein Entfernen des Kopfendes (111) vom Rückenende (121) zu verhindern, wenn sich die Klemme (10) in der geschlossenen Stellung befindet, wenn Druck nur auf das Kopfende (111) ausgeübt wird, wobei die Arretiermittel aus einem Zapfen (112), der sich am Kopfende (111) befindet, und aus einer Aussparung (122) bestehen, die sich am Rückenende (121) befindet, wobei der Zapfen (112) und die Aussparung (122) derart angeordnet sind, dass in der geschlossenen Stellung der Klemme der Zapfen (112) in die Aussparung (122) hineinreicht, wobei ein Betätigungsmittel (115) vorzugsweise am Kopfende (111) vorgesehen ist, **dadurch gekennzeichnet, dass** der Zapfen (112) und die Aussparung (122) jeweils mit einer Anlagefläche versehen sind, die derart zusammenwirken, dass der Zapfen und die Aussparung aneinander zur Anlage kommen, wenn versucht wird, das Kopfende (111) mit einer Bewegung im Wesentlichen senkrecht zur Eindringrichtung des Zapfens vom Rückenende (121) zu entfernen, wobei die Anlagefläche des Zapfens (112) und der Aussparung (122) derart im Wesentlichen senkrecht zur Überlappungsfläche der beiden Enden (111, 121) verlaufen, dass zum Öffnen der Klemme zuerst Druck auf den Rücken (12) ausgeübt werden muss, damit er in Richtung der Mitte der Klemme geschoben wird, sodass sich der Zapfen (112) aus der Aussparung (122) herausbewegt.

2. Klemme (10) nach dem vorhergehenden Anspruch, **dadurch gekennzeichnet, dass** ein Einsatz (15) in einer der Öffnungen (134) der Klemme (10) zum Aufnehmen des Endes des flexiblen Schlauchs vorgesehen ist, wobei der Einsatz (15) aus einem Stück mit der Klemme (10) gefertigt ist.

3. Klemme (10) nach Anspruch 2, **dadurch gekennzeichnet, dass** der Einsatz (15) mit einem Endstück (152) zum Befestigen eines weiteren flexiblen Schlauchs versehen ist.

4. Klemme (10) nach Anspruch 2 oder 3, **dadurch gekennzeichnet, dass** der Einsatz (15) an seinem Außenumfang mit einer Rille (151) versehen ist, in die die beiden gegenüberliegenden Ränder der Öffnung (134) der Klemme (10) hineinreichen.

5. Klemme (10) nach einem der vorhergehenden Ansprüche, **dadurch gekennzeichnet, dass** eine Längsrille (125) auf der Außenfläche des Rückens (12) vorgesehen ist.

6. Infusionsset, das aus einem flexiblen Schlauch besteht, der durch eine Klemme (10) nach Anspruch 1 bis 5 verläuft.

7. Set nach Anspruch 6, **dadurch gekennzeichnet, dass** zwei flexible Schläuche unterschiedlicher Beschaffenheit mittels eines Einsatzes (15), der aus einem Stück mit der Klemme (10) gefertigt ist, miteinander verbunden sind.

8. Kombination aus einer Infusionspumpe (20) und einem flexiblen Infusionsschlauchsatz nach Anspruch 6 oder 7, wobei die Infusionspumpe (20) mit einer Tür (22) versehen ist, die zum Einlegen oder Entnehmen eines flexiblen Schlauchs, der mit einer Klemme (10) nach einem der Ansprüche 1 bis 5 versehen ist, geöffnet und geschlossen werden kann, wobei die Pumpe mit Mitteln zum Öffnen oder Schließen einer Klemme (10) versehen ist, die einen schwenkbaren Hebel (21) umfassen, der mit mindestens einem Kopfnocken (211) versehen ist, um das Kopfende (111) vom Rückenende (121) einer in der Pumpe befindlichen Klemme (10) zu entfernen, **dadurch gekennzeichnet, dass** der Hebel (21) mit Mitteln (212) zum Entsperren der Arretiermittel (112, 122) der Klemme (10) versehen ist.

9. Kombination nach dem vorhergehenden Anspruch, **dadurch gekennzeichnet, dass** die Infusionspumpe mit Mitteln (211, 212) versehen ist zum Schließen der Klemme (10), wenn die Tür (22) geöffnet wird.

10. Kombination nach Anspruch 8 oder 9, **dadurch gekennzeichnet, dass** die Entsperrmittel aus einem Rückennocken (212) bestehen, wobei der Kopfnocken (211) und der Rückennocken (212) auf der Schwenkachse (213) des Hebels (21) derart angeordnet sind, dass beim Betätigen des Hebels (21) zum Öffnen der Klemme der Rückennocken (212) zuerst auf den Rücken (12) der Klemme (10) drückt, bis er die Arretierung der Arretiermittel (112, 122) der Klemme (10) löst und sie in dieser Stellung hält, dann der Kopfnocken (211) das Kopfende (111) wegdrückt, bis ein Durchgang für das Rückenende (121) geschaffen ist, der Rückennocken (212) anschließend den Druck auf den Rücken (12) der Klemme (10) wegnimmt und schließlich der Kopfnocken (211) das Kopfende (111) loslässt.

11. Kombination nach einem der Ansprüche 8 bis 10, **dadurch gekennzeichnet, dass** der Hebel (21) in der Tür (22) der Pumpe (20) platziert ist.

12. Kombination nach einem der Ansprüche 8 bis 11, **dadurch gekennzeichnet, dass** der Hebel (21) ferner mit einem Haken (24) versehen ist, der mit einer Rolle (231) zusammenwirken kann, die sich auf dem Gehäuse (23) der Pumpe befindet, damit die Tür (22) geschlossen gehalten wird.

13. Kombination nach dem vorhergehenden Anspruch, **dadurch gekennzeichnet, dass** die Tür (22) mit einem Rückstellmittel (25) versehen ist, das dazu dient, den Haken (24) in die geschlossene Stellung zu bringen, sobald die Mittel zum Öffnen und Schließen (211, 212) der Klemme (10) die Klemme (10) in die geöffnete Stellung gebracht haben.

14. Kombination nach einem der Ansprüche 8 bis 13, **dadurch gekennzeichnet, dass** eine Halterung (233) an dem Gehäuse (23) der Pumpe (20) zum Einrastenlassen des Einsatzes (15) der Klemme (10) vorgesehen ist.

## Claims

1. Clamp (10) that can be opened and reclosed for a flexible tube, notably for an infusion tube, consisting of an essentially U-shaped strip of plastic, the free end (111) of the first branch (11') of the U being bent over, to form the head (11) of the clamp, towards the free end (121) of the second branch (12), referred to as the back, so that in the closed position, the back end (121) of the back (12) is pressed underneath the free end (111) of the head (11), whereas in the open position, the free end (121) of the back (12) lies some distance above the free end (111) of the head (11), the clamp (10) having a pair of spaced-apart openings (114, 134) to allow a flexible tube to extend through the clamp (10) and to be supported thereby, and two opposing projections (113, 123) for closing off the flexible tube by compressing it when the clamp is in the closed position, means (115) being provided for parting the head end (111) from the back end (121) by applying pressure to the head end (111) parallel to the surfaces of overlap of the two ends so as to open the clamp when it is in the closed position, blocking means (112, 122) being provided to prevent the head end (111) from being parted from the back end (121) when the clamp (10) is in the closed position when only pressure is exerted on the head end (111), the blocking means consisting of a stud (112) situated on the head end (111) and of a recess (122) situated on the back end (121), the stud (112) and the recess (122) being arranged in such a way that when the clamp is in the closed position, the stud (112) enters the recess (122), an actuating element (115) preferably being provided on the head end (111), **characterized in that** the stud (112) and the recess (122) are each equipped with an abutment surface which surfaces collaborate with one another in such a way that the stud and the recess come into abutment against one another if an attempt is made to separate the head end (111) from the back end (121) in a movement substantially perpendicular to the direction of penetration of the stud, the abutment surfaces of the stud (112) and of the recess (122) being substantially perpendicular to the surface of overlap of the two ends (111, 121) so that, in order to open the clamp, it is necessary first of all to apply pressure to the back (12) in order to move it towards the centre of the clamp so as to cause the stud (112) to leave the recess (122).

2. Clamp (10) according to the preceding claim, **characterized in that** an insert (15) is provided in one of the openings (134) of the clamp (10) to accept the end of the flexible tube, the insert (15) being secured to the clamp (10).

3. Clamp (10) according to Claim 2, **characterized in that** the insert (15) is provided with an end piece (152) for attaching another flexible tube.

4. Clamp (10) according to Claim 2 or 3, **characterized in that** the insert (15) is equipped on its external periphery with a slot (151) that the two opposite edges of the opening (134) of the clamp (10) enter.

5. Clamp (10) according to one of the preceding claims, **characterized in that** a longitudinal slot (125) is provided on the exterior face of the back (12).

6. Infusion set consisting of a flexible tube passing through a clamp (10) according to Claims 1 to 5.

7. Set according to Claim 6, **characterized in that** two flexible tubes of different qualities are connected to one another by means of an insert (15) secured to the clamp (10).

8. Combination of an infusion pump (20) and of a flexible tube infusion set according to one of Claims 6 and 7, in which the infusion pump (20) is provided with a door (22) that can be opened and closed so as to fit or remove a flexible tube fitted with a clamp (10) according to one of Claims 1 to 5, the pump being fitted with means for opening or closing a clamp (10) which means comprise a pivoting lever (21) equipped with at least one head cam (211) for parting the head end (111) from the back end (121) of a clamp (10) situated in the pump, **characterized in that** the lever (21) is equipped with means (212) for unblocking the blocking means (112, 122) of the clamp (10).

9. Combination according to the preceding claim, **characterized in that** the infusion pump is equipped with means (211, 212) for closing the clamp (10) when the door (22) is opened.

10. Combination according to Claim 8 or 9, **characterized in that** the unblocking means consist of a back cam (212), the head cam (211) and the back cam (212) being arranged on the pivot axle (213) of the lever (21) in such a way that as the lever (21) is actuated to open the clamp, the back cam (212) presses first of all against the back (12) of the clamp (10) until the blocking means (112, 122) of the clamp (10) are unlocked, and keeps it in this position, then the head cam (211) moves the head end (111) away until a passage is left for the back end (121), the back cam (212) then releases the pressure on the back (12) of the clamp (10), and finally, the head cam (211) releases the head end (111).

11. Combination according to one of Claims 8 to 10, **characterized in that** the lever (21) is placed in the door (22) of the pump (20).

12. Combination according to one of Claims 8 to 11, **characterized in that** the lever (21) is further equipped with a hook (24) capable of collaborating with a roller (231) situated on the housing (23) of the pump for keeping the door (22) closed.

13. Combination according to the preceding claim, **characterized in that** the door (22) is equipped with a return element (25) which tends to move the hook (24) into the closed position as soon as the means (211, 212) of opening and closing the clamp (10) have placed the clamp (10) in the open position.

14. Combination according to one of Claims 8 to 13, **characterized in that** a support (233) is provided on the housing (23) of the pump (20) to clip onto the insert (15) of the clamp (10).
